# EUROPÄISCHE PATENTSCHRIFT

(11) **EP 0 530 400 B1**
(45) Veröffentlichungstag und Bekanntmachung des Hinweises auf die Patenterteilung: **18.12.1996**
(21) Anmeldenummer: 91115001.9
(22) Anmeldetag: 05.09.1991
(51) Int. Cl.: A61B 17/39

(54) **Instrument für die Hochfrequenzchirurgie zum Schneiden und/oder Koagulieren mit HF-Strom**
H.F.-Surgical instrument for cutting and coagulating
Instrument chirurgical à H.F. pour couper et coaguler

(43) Veröffentlichungstag der Anmeldung: 10.03.1993
(73) Patentinhaber: Erbe Elektromedizin GmbH., D-72072 Tübingen (DE)
(72) Erfinder: Farin, Günter, W-7400 Tübingen-Hirschau (DE); Fischer, Klaus, W-7270 Nagold-Emmingen (DE); Buess, Gerhard, Prof. Dr. med., W-7400 Tübingen (DE)
(74) Vertreter: Endlich, Fritz, Dipl.-Phys. Patentanwalt

(56) Entgegenhaltungen:
- FR-A- 2 235 669
- US-A- 2 056 377
- US-A- 4 565 200
- US-A- 5 007 908
- US-A- 5 009 656

## Beschreibung

Die Erfindung betrifft ein Instrument für die Hochfrequenzchirurgie zum Schneiden und/oder Koagulieren biologischer Gewebe mittels hochfrequenten elektrischen Wechselstroms, im folgenden kurz HF-Strom genannt.

Aus der US-A-5,007,908 ist ein Instrument entsprechend dem Oberbegriff des Patentanspruches 1 bekannt.

Aus US-A-4,043,342, Figuren 42 bis 47, ist ein sesquipolares, elektrochirurgisches Instrument bekannt, bei welcher eine dünne Nadel als aktive Schneideelektrode starr in einer als Neutralelektrode wirkenden Metallhülse angeordnet ist, wobei der HF-Strom während Schneidevorgängen zwischen der Nadel und der Metallhülse durch das zu schneidende Gewebe fließt. Eine weitere Ausgestaltung dieses sesquipolaren Instruments ist in der selben Patentschrift anhand der Figuren 45 bis 49 beschrieben. Hierbei ist die Nadel in der Metallhülse so angeordnet, daß sie im Ruhezustand durch einen Federmechanismus in die Metallhülse zurückgezogen ist. Wird beim Schneiden mit einem derartigen Instrument eine Kraft in axialer Richtung auf die als Neutralelektrode wirkende Hülse ausgeübt, so kann die Tiefe der Schnittführung durch die Kraft variiert werden. Dieses Instrument ist jedoch für endoskopische Operationen nicht geeignet, weil bei endoskopischen Operationen in der Regel die Freiheitsgrade der räumlichen Bewegungen der Operationsinstrumente stark begrenzt sind. Außerdem können bei endoskopischen Operationen die in den Figuren 45 bis 49 dargestellten Elektroden nicht angewendet werden, weil die zum Herausschieben der Nadelelektrode erforderliche Kraft infolge der in der Regel weichen Gewebestrukturen und begrenzten Bewegungsfreiheit nicht ausreichend kontrolliert realisiert werden kann.

Die aus US-A-4,043,342, Figuren 42 bis 49, bekannten Instrumente sind zwar zum Schneiden, nicht jedoch zum Koagulieren geeignet, weswegen ein Wechseln verschiedener Instrumente während einer Operation erforderlich ist.

Aus DE 34 23 356 C2 ist ein elektrochirurgisches Hochfrequenz-Schneideinstrument bekannt, welches nicht nur zum Schneiden, sondern auch zum bipolaren Koagulieren geeignet sein soll.

Dieses Schneideinstrument ist jedoch für endoskopische Anwendungen nicht geeignet, weil, ähnlich wie beim US-A-4,043,342, die Freiheitsgrade der Schnittführung, und dies gilt in gleicher Weise auch für die Koagulation, infolge der axialen Ausrichtung sowohl der Nadelelektrode als auch der bipolaren Koagulationselektrode stark begrenzt ist.

Die endoskopische Chirurgie ist dadurch gekennzeichnet, daß Operationsfelder innerhalb des Körpers durch anatomisch gegebene oder minimal invasiv angelegte Zugänge erreicht werden. Dies ist ein großer Vorteil für den Patienten, weil die bei offenen Operationstechniken erforderlichen invasiven Eröffnungen der betreffenden Körperhöhlen vermieden werden. Der Operateur hat hierbei in der Regel jedoch keine direkte Sicht auf das Operationsfeld, sondern muß optische und/oder elektronische Bildübertragungssysteme verwenden. Da die Operationsinstrumente hierbei durch relativ enge Öffnungen und/oder Kanäle in den Körper eingeführt werden, ist die Bewegunesfreiheit für die Operationsinstrumente im Vergleich zu offenen chirurgischen Operationsmethoden mehr oder weniger begrenzt. Außerdem kann das Wechseln der für eine Operation erforderlichen Instrumente sehr zeitraubend sein. Dies betrifft auch Instrumente zum Schneiden und/oder Koagulieren mittels HF-Strom. Ein besonderes Problem, insbesondere bei endoskopischen Operationen, stellt die Blutstillung dar. Beim Präparieren und/oder Trennen von Gewebestrukturen entstehen unvermeidlich mehr oder weniger starke Blutungen, welche die Übersicht über das Operationsfeld stören oder gar völlig verdecken. Der Operateur muß bei derartigen Komplikationen das Blut mittels geeigneter Saugeinrichtungen aus dem Operationsfeld absaugen und die Blutungsquelle beispielsweise durch Koagulation stillen. Es sind spezielle Saugrohre bekannt, weiche auch als Koagulationselektrode verwendet werden können. Zum Schneiden sind diese Saugrohre jedoch nicht geeignet, so daß ein Wechseln verschiedener Instrumente während einer Operation erforderlich ist.

Da innerhalb des Körpers in der Regel feine Gewebestrukturen präpariert und reseziert werden müssen, sollen die zum Schneiden und/oder Koagulieren erforderlichen HF-Ströme möglichst klein sein, da andernfalls unbeabsichtigte thermische Schädigungen lateraler Gewebestrukturen entstehen können.

Die zum Schneiden biologischer Gewebe erforderlichen HF-Ströme sind weitgehend proportional dem geometrischen Querschnitt der Schneideelektrode in Schnittrichtung. Mit Rücksicht auf unerwünschte Nebeneffekte, z.B. thermische Schädigung der Schnittränder, sollte die Schneideelektrode möglichst dünn sein. Diese Forderung wird beispielsweise von einer dünnen Nadelelektrode erfüllt. Je dünner eine Nadelelektrode ist, desto größer wird andererseits das Risiko, daß sie versehendlich verbogen oder gar abgebrochen wird. Außerdem besteht bei dünnen Elektroden, insbesondere bei dünnen Nadelelektroden, Verletzungsgefahr sowohl für den Patienten als auch für das Personal.

Je dünner eine Nadelelektrode ist, desto weniger ist sie zur Blutstillung durch thermische Koagulation der Blutungsquelle mit HF-Strom geeignet, weil beim direkten Berühren der Blutungsquelle mit einer dünnen Nadelelektrode die effektive Kontaktfläche zu klein ist, um eine suffiziente Koagulationstiefe zu erreichen. Beim indirekten Berühren der Blutungsquelle über ein anderes elektrisch leitfähiges Instrument, z.B. eine metallische Klemme, besteht das Risiko, daß dünne Schneideelektroden verbiegen oder, infolge zu großen HF-Stroms, verglühen. Beim indirekten Koagulieren über ein anderes elektrisch leilfähiges Instrument, besteht insbesondere bei endoskopischen Operationen das Risiko, daß das zum Koagulieren dienende Instrument wesentlich mehr Gewebe berührt als für eine suffiziente Blutstillung erforderlich, und dadurch mehr Gewebe koaguliert wird als erforderlich.

Es ist Aufgabe der Erfindung, ein chirurgisches Instrument zum Schneiden und/oder Koagulieren biologischer Gewebe mit möglichst kleinen HF-Strömen so zu gestalten, daß es in der endoskopischen Chirurgie verwendbar ist und es ermöglicht, daß, auch bei begrenzten Freiheitsgraden der Bewegung des Instruments, insbesondere räumliche Präparationsschnitte und Koagulationen durchführbar sind.

Diese Aufgabe wird erfindunasgemäß durch den Gegenstand des Anspruches 1 gelöst. Weitere Ausgestaltungen der Erfindung sind Gegenstand der Unteransprüche.

Ein derartiges Instrument zum Schneiden und/oder Koagulieren mit HF-Strom, insbesondere für endoskopische Operationen enthält, ähnlich wie das in Figur 45 von US-A-4,043,342 dargestellte sesquipolare elektrorochirurgische Instrument, eine elektrisch leitfähigen Hülse, aus deren Ende eine elektrisch leitfähige Nadelelektrode als Schneideelektrode herausragt, welche innerhalb der Hülse gegen diese Hülse elektrisch isoliert und derart verschiebbar ist, daß sie vollständig in die Hülse zuückgezogen werden kann. Um die Freiheitsgrade der Schnittführung und/oder Koagulation, insbesondere bei endoskopischer Anwendung, z.B. bei der laparoskopischen Cholecystektomie, bei der transanalen endoskopischen Mikrochirurgie oder bei der Adhäsiolyse flexibler zu gestalten, ist die Hülse inklusive Nadelelektrode und elektrischer Isolation zwischen Nadelelektrode und Hülse kurz vor dem zum Patienten gerichteten Ende des Instruments in einem für den jeweiligen Verwendungszweck geeigneten Winkel abgebogen. Die Hülse ist an ihrem proximalen Ende mit einem Schaft ausgestattet, welcher aus einem Metallrohr besteht und eine dem jeweiligen endoskopischen Verwendungszweck entsprechende Länge von beispielsweise 20 bis 50 cm und einen Durchmesser von beispielsweise 5 Millimetern hat. Außerdem ist die Außenfläche der Hülse und des Schafts außen, bis auf die zum Patienten gerichtete Stirnfläche der Hülse, mit einer elektrischen Isolierschicht ausgestattet. Die elektrisch nicht isolierte Stirnfläche der Hülse dient als Koagulationselektrode. Die Hülse besteht beispielsweise aus einem Metallrohr, welches an der Stimfläche konvex, beispielsweise halbkugelförmig, bis auf ein kleines Loch, aus welchem die Nadelelektrode herausragt, geschlossen ist. Hülse und Schaft können auch einstückig aus einem Rohr geformt sein, wobei die Durchmesser von Hülse und Schaft ungleich oder auch gleich sein können. Der Durchmesser der Hülse sollte mit Rücksicht auf eine ungestörte Sicht auf die Nadelelektrode möglichst dünn sein. Der Durchmesser des Schaftes sollte mit Rücksicht auf die mechanische Stabilität des Instrumentes möglichst groß sein.

In einer Ausgestaltung der Erfindung ist die Hülse und/oder der Schaft des Instrumentes plastisch verbiegbar, so daß der Operateur das Instrument der jeweiligen anatomischen Situation anpassen kann.

Die zum Schneiden dienende Nadelelektrode kann manuell oder mittels eines elektromagnetischen, pneumatischen oder hydraulischen Antriebs zum Schutz gegen Verbiegen oder Abbrechen oder gegen unbeabsichtigte Verletzung des Patienten oder während Koagulationen, für die nur die Stirnfläche der Hülse verwendet wird, in die Hülse zurückgezogen und zum Schneiden wieder aus der Hülse herausgeschoben werden. Hierzu können auch mechanische Einrichtungen, wie sie beispielsweise in vielfältiger Ausführung bei Kugelschreibern zum rastenden Vor- und Rückführen bzw. zum Arretieren der Schreibmine bekannt sind, verwendet werden.

In einer weiteren Ausgestaltung kann das erfindungsgemäße Instrument mit einer Vorrichtung ausgestattet werden, an welcher die zum Schneiden effektiver Länge der Nadelelektrode einstellbar ist.

Der Antrieb für die Verschiebung der zum Schneiden dienenden Nadelelektrode ist beispielsweise am Ende des Schaftes angeordnet, an welchem auch die elektrischen Anschlüsse für die zum Schneiden dienende Nadelelektrode und für die als Koagulationselektrode oder Neutralelektrode dienende Stirnfläche der Hülse angeordnet sein können.

Die Erfindung wird im folgenden anhand schematischer Zeichnungen und Ausführungsbeispielen detaillierter beschrieben. Es zeigen:
- Figur 1: eine schematische Darstellung tumoröser Veränderungen im Rektum.
- Figur 2: eine schematische Darstellung einer Anwendung eines erfindungsgemäßen Instruments
- Figur 3: eine Schnittdarstellung des distalen Endes des Instrumentes, und zwar mit herausgeschobener Nadelelektrode in Figur 3a und mit hereingezogener Nadelelektrode in Figur 3b.
- Figur 4: eine schematische Darstellung eines Ausführungsbeispiels eines erfindungsgemäßen Instruments
- Figur 5: eine schematische Darstellung eines Antriebs für die Schneideelektrode, und zwar eines elektromagnetischen Antriebs in Figur 5a und eines pneumatischen Antriebs in Figur 5b
- Figur 6: eine Ausgestaltung des erfindungsgemäßen Instruments. Hierbei ist der Schaft des Instruments plastisch verbiegbar gestaltet, so daß der Operateur das Instrument der jeweiligen anatomischen Situation anpassen kann.
- In Figur 7: ist ein Ausführungsbeispiel einer weiteren Ausgestaltung des erfindungsaemäßen Instruments mit einer Einrichtung zum Einstellen der zum Schneiden effektiven Länge der Nadelelektrode dargestellt, und zwar in einer Schnittdarstellung 7a und einer Außenansicht 7b.
- Figur 8: Zeigt in schematischer Darstellung ein Operationsrektoskop

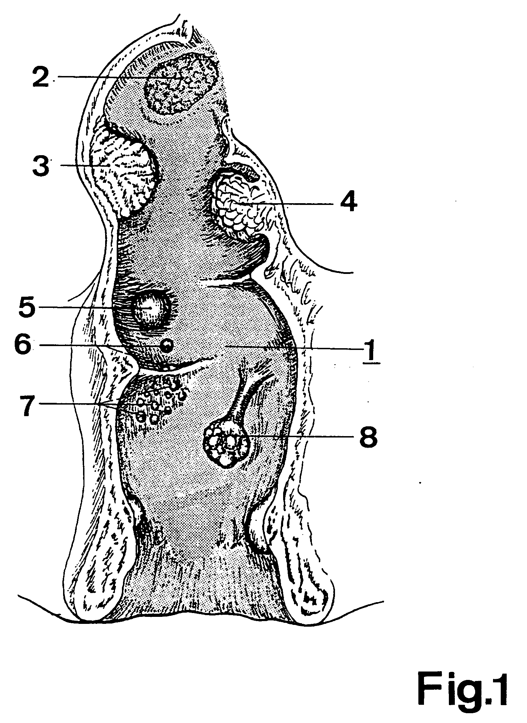

Figur 1 zeigt Beispiele von tumorösen Veränderungen beispielsweise im Rektum 1, die mit einem erfindungsgemäßen Instrument operativ behandelt werden können, und zwar ulzerierende Karzinome 2, villöse Adenome 3, polypoide Karzinome 4, sessile adenomatöse Polypen 5, 6, 7 und/oder gestielte adenomatöse Polypen 8. Bei Verwendung von bisher bekannten Instrumenten können derartige tumoröse Veränderungen nicht so präzise und nicht ohne ständiges Wechseln von Schneide und Koagulationsinstrumenten reseziert werden.

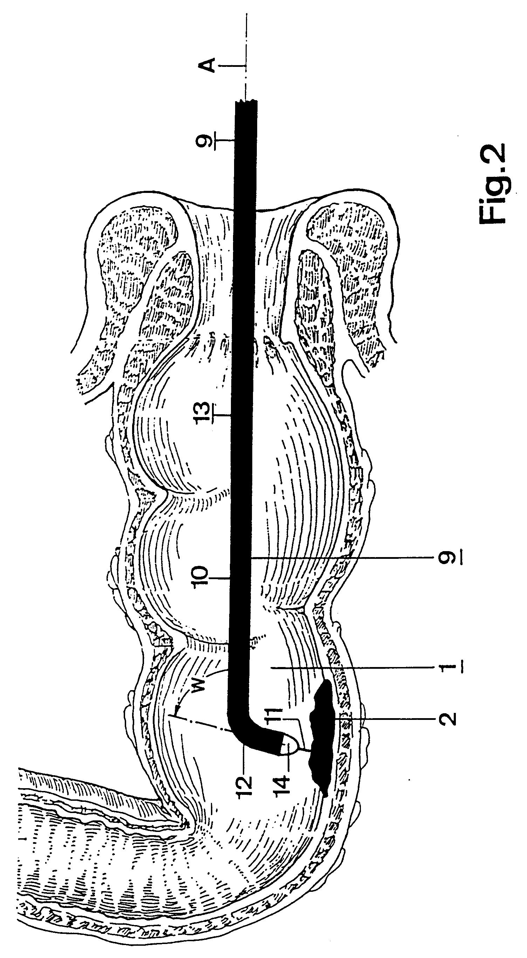

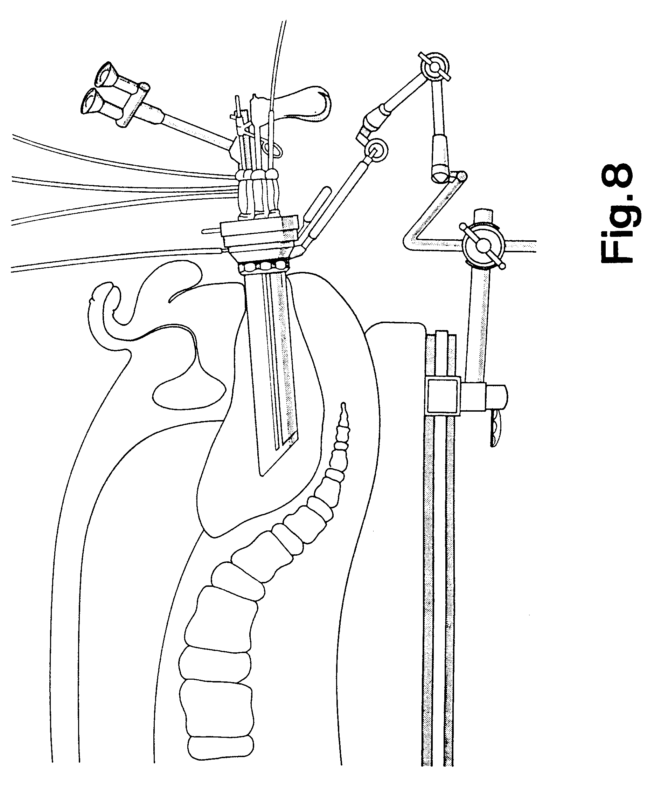

Anhand von Figur 2 wird schematisch beispielsweise eine transanale Anwendung eines erfindungsgemäßen Instruments zur operativen Therapie eines Karzinoms im Rektum 1 dargestellt, wobei mit Rücksicht auf eine übersichtliche Darstellung nur das zum Patienten proximale Teil des Instruments 9 dargestellt ist. Das Instrument 9 ist transanal ins Rektum 1 eingeführt. In der Praxis erfolgt die Anwendung des Instruments durch ein geeignetes Operationsrektoskop, wie es in Figur 8 schematisch dargestellt ist. Das Instrument 9 ist bis zum abzutragenden Karzinom 2 eingeführt. Zum Resezieren des Karzinoms 2 ist die Nadelelektrode 11 aus dem Instrument 9 herausgeschoben. Wird während der Resektion ein Blutgefäß perforiert, so kann es mit dem als Koagulationselektrode des zum Patienten proximalen Endes der Hülse 12 bzw. des Instruments 9 koaguliert werden. Während einer Koagulation kann die Nadelelektrode 11 in das Instrument 9 zurückgezogen werden. Das zum Patienten proximale Ende der Hülse 12 bzw. des Instruments 9 ist gegen die Achse A des Schafts 13 in einem für den jeweiligen Anwendungszweck geeigneten Winkel W abgebogen. Der Schaft 13 und die Hülse 12 sind auf ihrer Außenfäche, exklusive der als Koagulationselektrode ausgebildeten konvexen Stirnfläche 14, mit einer elektrischen Isolation 10 ausgestattet.

Die Nadelelektrode 11 kann als monopolare Schneideelektrode betrieben werden, wobei als Gegenelektrode eine bei monopolaren Techniken übliche, bekannte Neutralelektrode in bekannter Weise auf der Haut des Patienten appliziert ist. Hierbei wird die Nadelelektrode 11 an die monopolare, aktive Ausgangsbuchse und die Neutralelektrode an die neutrale Ausgangsbuchse des Hochfrequenzgenerators angeschlossen. Als Gegenelektrode kann aber auch die Stirnfläche 14 der Hülse 12 verwendet werden, wie es in US-A-4,043,342 beschrieben ist. Hierbei wird die Nadelelektrode 11 an die monopolare, aktive Ausgangsbuchse und die Stirnfläche 14 der Hülse 12 an die neutrale Ausgangsbuchse des Hochfrequenzgenerators angeschlossen.

Die Stirnfläche 14 der Hülse 12 kann als monopolare Koagulationselektrode betrieben werden, wobei als Gegenelektrode eine bei monopolaren Techniken übliche, bekannte Neutralelektrode in bekannter Weise auf der Haut des Patienten appliziert ist. Hierbei wird die als Koagulationselektrode dienende Stirnfläche 14 der Hülse 12 an die monopolare, aktive Ausgangsbuchse und die auf der Haut des Patienten applizierte Neutralelektrode an der neutralen Ausgangsbuchse des Hochfrequenzgenerators angeschlossen.

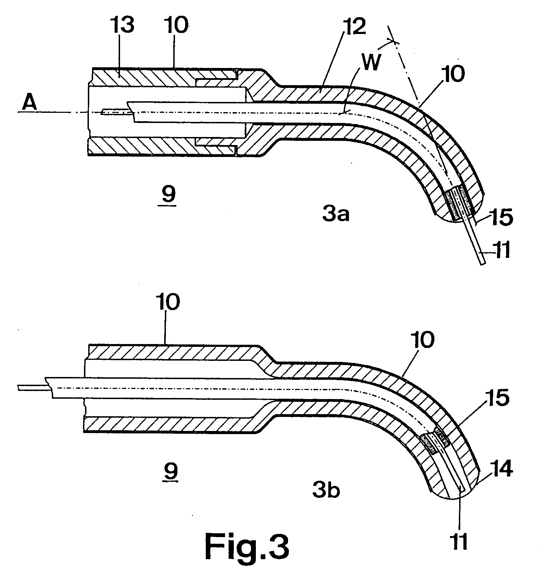

In Figur 3 ist das distale, abgebogene Ende der Hülse 12 bzw. des Instruments 9 im Schnitt dargestellt, und zwar mit herausgeschobener Nadelelektrode 11 zum Schneiden in Figur 3a und mit hereingezogener Nadelelektrode 11 zum Koagulieren bzw. in Ruhestellung in Figur 3b. In Figur 3a ist die Hülse 12 in den Schaft 13 eingefügt. In Figur 3b sind Hülse und Schaft einstückig aus einem Rohr geformt. Die Außendurchmesser der Hülse 12 und des Schaftes 13 können prinzipiell gleich sein. Mit Rücksicht auf eine gute Sicht auf die Nadelelektrode 11 und eine nicht zu große effeklive Kontaktfläche der als Koagulationselektrode verwendbaren, vorzugsweise konvexen Stirnfläche 14 der Hülse 12 ist es jedoch zweckmäßig, den Außendurchmesser der Hülse 12 in der Nähe der Stirnfläche 14 möglichst klein, beispielsweise 3 Millimeter zu wählen. Mit Rücksicht auf die mechanische Stabilität des Instruments ist es zweckmäßig, den Außendurchmesser des Schaftes 13 nicht zu klein zu wählen, beispielsweise 5 Millimeter. Der Außendurchmesser der Hülse 12 kann zweckmäßig in mindestens einer Stufe, wie in Figur 3 dargestellt, und/oder konisch vom Außendurchmesser des Schaftes 13 auf den Durchmesser der Stirnfläche 14 reduziert werden.

Die Nadelelektrode 11 ist mit einer elektrischen Isolation 15 gegen die metallische Hülse 12 bzw. den Schaft 13 isoliert. Schaft 13 und Hülse 12 sind auf ihrer gesamten Oberfläche, exclusive der als Koagulationselektrode dienenden Stirnfläche 14 der Hülse 12, mit einer elektrischen Isolation 10 ausgestattet.

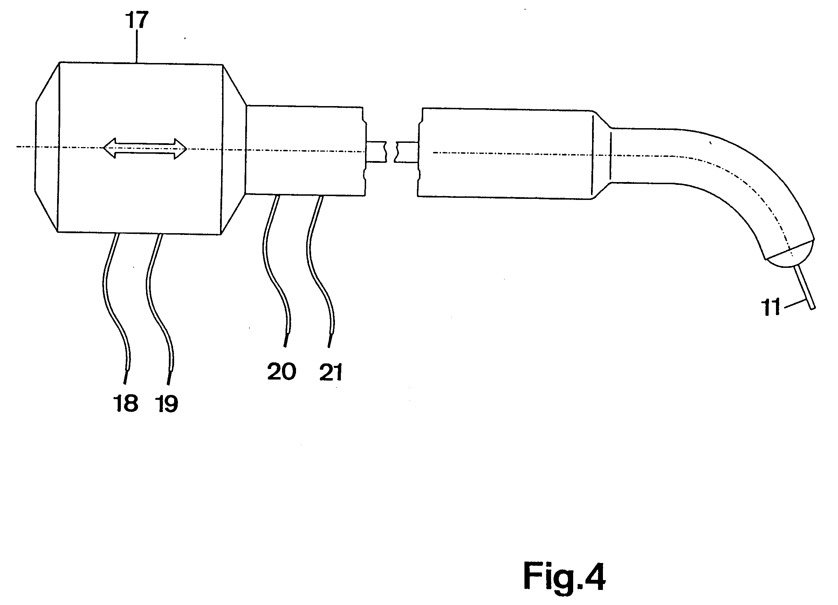

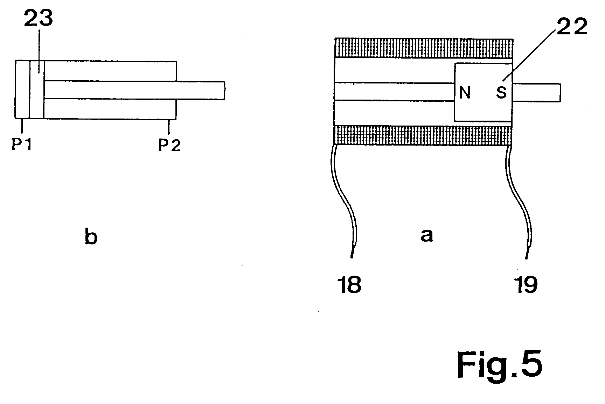

In Figur 4 ist schematisch ein Ausführungsbeispiel eines erfindungsgemäßen Instruments dargestellt. Am proximalen Ende des Instruments ist ein Antrieb 17 zum Hinein- bzw Herausschieben der Nadelelektrode 11 schematisch dargestellt. Dieser Antrieb 17 kann beispielsweise elektromagnetisch, wie in Figur 5a dargestellt, oder pneumatisch oder hydraulisch, wie in Figur 5b dargestellt, oder mechanisch, wie beispielsweise bei Kugelschreibern zum Heraus- und Hineinbewegen der Schreibmine bekannt, realisiert werden.

Die Positionen 18 und 19 sind die Anschlüsse für den elektromagnetischen Antrieb. Bei pneumatischem oder hydraulischem Antrieb müßten diese Anschlüsse P1 und P2 Rohre oder Schläuche sein. 20 ist beispielsweise der elektrische Anschluß für die Nadelelektrode, 11 und 21 der elektrische Anschluß für die als Koagulationselektrode oder Neutralelektrode wirkende Stirnfläche 14 der Hülse 12.

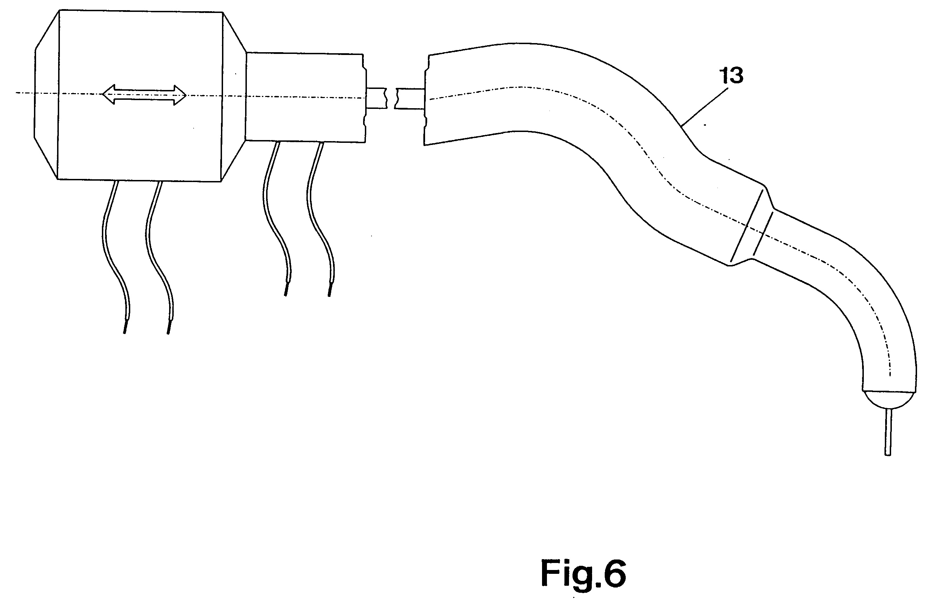

In Figur 6 ist ein erfindungsgemäßes Instrument schematisch dargestellt, bei welchem der Schaft 13 vom Operateur plastisch so gebogen werden kann, daß es den individuellen bzw. anatomischen Verhältnissen angepaßt werden kann.

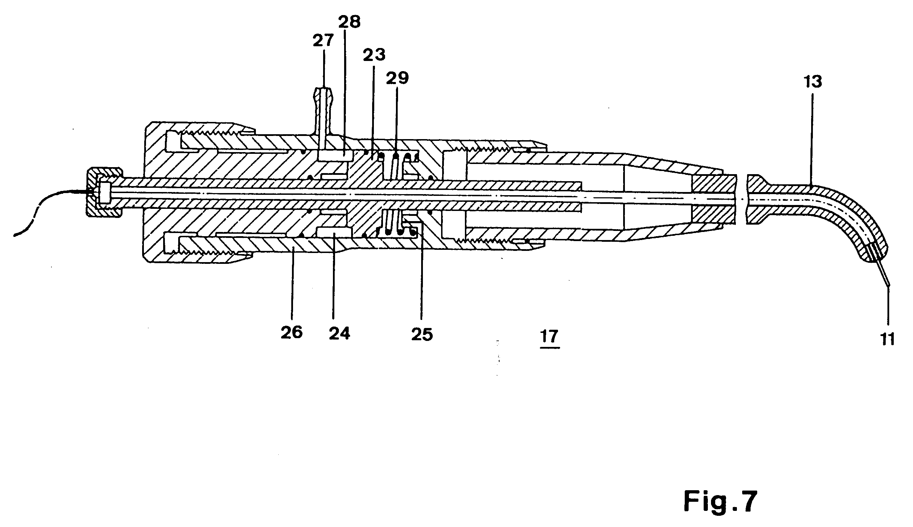

In Figur 7 ist ein Ausführungsbeispiel einer weiteren Ausgestaltung eines erfindungsgemäßen Instruments mit einer Einrichtung zum Einstellen der zum Schneiden effektiven Länge der Nadelelektrode dargestellt, anhand eines pneumatischen Antriebs in einer Schnittdarstellung. Die Nadelelektrode 11 ist mit dem Kolben 23 des pneumatischen Antriebs 17 mechanisch gekoppelt. Der maximale Hub des Kolbens 23 wird durch zwei Anschläge 24 und 25 begrenzt. Anschlag 24 definiert die Stellung der distalen Spitze der Nadelelektrode im hineingezogenen Zustand. Die relative Stellung der zum Patienten proximalen Spitze der Nadelelektrode zum proximalen Ende des Instruments kann beispielsweise durch mehr oder weniger tiefes Hereinschrauben des Schafts 13 in den Zylinder 26 des Antriebs 17 justiert werden. Der maximale Hub des Kolbens 23 kann beispielsweise durch mehr oder weniger tiefes Hineinschrauben des Anschlags 24 in den Zylinder 26 justiert werden. Am Schlauchanschluß 27 wird zum Herausschieben der Nadelelektrode Gas oder Flüssigkeit in den Kolbenraum 28 mit Druck eingeleitet. Eine Druckfeder 29 schiebt die Nadelelektrode in das Instrument hinein, wenn kein Druck im Kolbenraum vorhanden ist.

Figur 8 zeigt in schematischer Darstellung ein Operationsrektoskop zur transanalen endoskopischen Resektion tumoröser Veränderungen im Rektum, durch welches hindurch das erfindungsgemäße Instrument angewendet werden kann.

## Patentansprüche

1. Instrument für die Hochfrequenzchirurgie zum Schneiden und/oder Koagulieren biologischer Gewebe zum Einsetzen in ein Endoskop zur Durchführung endoskopischer Operationen, mit einem auf seiner Außenfläche mit einer elektrischen Isolation (10) ausgestatteten Schaftelement, dessen distales Endstück eine metallische Hülse (12) bildet, worin eine als Schneidelektrode dienende metallische Nadel (11) um einen Winkel (W) aus der axialen Richtung (A) des proximalen Endstücks des Schaftelements abgebogen und herausschiebbar angeordnet ist, die gegen die Hülse (12) elektrisch isoliert ist,
**dadurch gekennzeichnet,** daß
sich die elektrische Isolation (10) auch über die Außenfläche der Hülse (12) mit Ausnahme einer als Koagulationselektrode oder als Neutralelektrode dienenden distalen Stirnfläche (14) erstreckt, und
daß das distale Ende der Hülse (12) mit der Nadelelektrode (11) um den Winkel (W) abgebogen ist, wobei die Nadelelektrode (11) in axialer Richtung herausschiebbar angeordnet ist.

2. Instrument nach Anspruch 1,
dadurch gekennzeichnet,
daß die Nadelelektrode (11) als monopolare Schneideelektrode dient, wobei als Gegenelektrode eine Neutralelektrode auf der Haut des Patienten appliziert ist.

3. Instrument nach Anspruch 1,
dadurch gekennzeichnet,
daß die Stirnfläche (14) der Hülse (12) als monopolare Koagulationselektrode dient, wobei als Gegenelektrode eine Neutralelektrode auf der Haut des Patienten appliziert ist.

4. Instrument nach Anspruch 1,
dadurch gekennzeichnet,
daß die Nadelelektrode (11) als Schneideelektrode und die Stirnfläche (14) der Hülse (12) als Neutralelektrode dient.

5. Instrument nach einem der vorhergehenden Ansprüche,
dadurch gekennzeichnet,
daß die Stirnfläche (14) der Hülse (12) konvex geformt ist.

6. Instrument nach einem der vorhergehenden Ansprüche,
dadurch gekennzeichnet,
daß vorzugsweise am proximalen Ende des Schaftelementes (13) ein Antrieb (17) zum Hineinziehen bzw. Herausschieben der Nadelelektrode (11) angeordnet ist.

7. Instrument nach Anspruch 6,
dadurch gekennzeichnet,
daß der Antrieb (17) zum Hineinziehen bzw. Herausschieben der Nadelelektrode (11) in bzw. aus der Hülse (12) elektromagnetisch erfolgt.

8. Instrument nach Anspruch 6,
dadurch gekennzeichnet
daß der Antrieb (17) zum Hineinziehen bzw. Herausschieben der Nadelelektrode (11) in bzw. aus der Hülse (12) pneumatisch oder hydraulisch erfolgt.

9. Instrument nach Anspruch 6,
dadurch gekennzeichnet,
daß das Instrument mit einer mechanische Einrichtung (17) zum Arretieren der Nadelelektrode (11) in der hineingezogenen (Fig. 3b) oder herausgeschobenen (Fig. 3a) Position ausgestattet ist.

10. Instrument nach einem der vorhergehenden Ansprüche,
dadurch gekennzeichnet,
daß die Länge der aus der Hülse (12) herausschiebbaren Nadelelektrode (11) einstellbar ist.

11. Instrument nach einem der vorhergehenden Ansprüche,
dadurch gekennzeichnet,
daß die Nadelelektrode einen Durchmesser von 0,1 bis 0,5 Millimeter hat.

12. Instrument nach einem der vorhergehenden Ansprüche,
dadurch gekennzeichnet,
daß das Schaftelement plastisch biegbar ist.

13. Instrument nach einem der vorhergehenden Ansprüche,
dadurch gekennzeichnet,
daß das Schaftelement eine Länge von 20 bis 50 cm hat.

14. Instrument nach einem der vorhergehenden Ansprüche,
dadurch gekennzeichnet,
daß die Hülse (12) und das Schaftelement einstückig sind.

15. Instrument nach einem der vorhergehenden Ansprüche,
dadurch gekennzeichnet,
daß die Hülse (12) und das Schaftelement gleiche Außendurchmesser haben.

16. Instrument nach einem der vorhergehenden Ansprüche,
dadurch gekennzeichnet,
daß der Außendurchmesser der Hülse (12) in der Nähe der Stirnfläche (14) kleiner ist als der Außendurchmesser des Schaftelementes.

17. Instrument nach Anspruch 16,
dadurch gekennzeichnet
daß der Außendurchmesser des Schaftelementes 5 bis 10 mm und der Außendurchmesser der Stirnfläche (14) der Hülse (12) 2 bis 5 mm sind.

18. Instrument nach Anspruch 16,
dadurch gekennzeichnet
daß der Außendurchmesser des Schaftelementes in mindestens einer Stufe oder konisch auf einen kleineren Außendurchmesser der Stimfläche (14) der Hülse (12) reduziert ist.

## Claims

1. An instrument for high frequency surgery for the cutting and/or coagulating of biological tissue for insertion into an endoscope to carry out endoscopic operations, with a shaft element equipped on its outer surface with an electrical insulation (10), the distal end piece of which forms a metallic sleeve (12) wherein a metallic needle (11), serving as a cutting electrode, bent about an angle (W) from the axial direction (A) of the proximal end piece of the shaft element is arranged, so as to be able to be pushed out, which needle (11) is electrically insulated with respect to the sleeve (12),
characterised in that
the electrical insulation (10) also extends over the outer surface of the sleeve (12) with the exception of a distal end face (14) serving as coagulation electrode or as neutral electrode, and
in that the distal end of the sleeve (12) with the needle electrode (11) is bent about the angle (W), in which the needle electrode (11) is arranged so as to be able to be pushed out in axial direction.

2. An instrument according to Claim 1,
characterised in that
the needle electrode (11) serves as monopolar cutting electrode, in which as counter electrode a neutral electrode is applied on the skin of the patient.

3. An instrument according to Claim 1,
characterised in that
the end face (14) of the sleeve (12) serves as monopolar coagulation electrode, in which as counter electrode a neutral electrode is applied on the skin of the patient.

4. An instrument according to Claim 1,
characterised in that
the needle electrode (11) serves as cutting electrode and the end face (14) of the sleeve (12) serves as neutral electrode.

5. An instrument according to one of the preceding Claims,
characterised in that
the end face (14) of the sleeve (12) is convex in shape.

6. An instrument according to one of the preceding Claims,
characterised in that
preferably at the proximal end of the shaft element (13) a drive (17) is arranged for drawing in or pushing out the needle electrode (11).

7. An instrument according to Claim 6,
characterised in that
the drive (17) for drawing in or pushing out the needle electrode (11) into or out from the sleeve (12) takes place electromagnetically.

8. An instrument according to Claim 6,
characterised in that
the drive (17) for drawing in or pushing out the needle electrode (11) into or out from the sleeve (12) takes place pneumatically or hydraulically.

9. An instrument according to Claim 6,
characterised in that
the instrument is equipped with a mechanical device (17) for arresting the needle electrode (11) in the drawn-in position (Fig. 3b) or pushed out position (Fig. 3a).

10. An instrument according to one of the preceding Claims,
characterised in that
the length of the needle electrode (11), which is able to be pushed out from the sleeve (12), is adjustable.

11. An instrument according to one of the preceding Claims,
characterised in that
the needle electrode has a diameter of 0.1 to 0.5 millimetres.

12. An instrument according to one of the preceding Claims.
characterised in that
the shaft element is able to be bent in a plastic manner.

13. An instrument according to one of the preceding Claims,
characterised in that
the shaft element has a length of 20 to 50 cm.

14. An instrument according to one of the preceding Claims,
characterised in that
the sleeve (12) and the shaft element are in one piece.

15. An instrument according to one of the preceding Claims,
characterised in that
the sleeve (12) and the shaft element have identical external diameters.

16. An instrument according to one of the preceding Claims,
characterised in that
the external diameter of the sleeve (12) in the vicinity of the end face (14) is smaller than the external diameter of the shaft element.

17. An instrument according to Claim 16,
characterised in that
the external diameter of the shaft element is 5 to 10 mm and the external diameter of the end face (14) of the sleeve (12) is 2 to 5 mm.

18. An instrument according to Claim 16,
characterised in that
the external diameter of the shaft element is reduced in at least one step or conically to a smaller external diameter of the end face (14) of the sleeve (12).

## Revendications

1. Instrument de chirurgie à haute fréquence destiné à la dissection et/ou à la coagulation de tissus biologiques et destiné à être introduit dans un endoscope afin de réaliser des opérations endoscopiques, possédant un élément de tige muni sur sa surface externe d'une isolation électrique (10) et dont la partie d'extrémité distale forme une douille métallique (12) dans laquelle s'engage, de manière à pouvoir être sortie, une aiguille métallique (11) servant d'électrode de dissection, cette aiguille étant écartée d'un angle (W) par rapport à la direction axiale de la partie d'extrémité proximale de l'élément de tige et étant isolée électriquement par rapport à la douille métallique (12),
caractérisé en ce que l'isolation électrique (10) s'étend également sur la surface externe de la douille (12), à l'exception d'une surface frontale distale (14) servant d'électrode de coagulation ou d'électrode neutre, et en ce que l'extrémité distale de la douille (12) avec l'aiguille électrode (11), est écartée de l'angle (W), ladite aiguille électrode (11) étant disposée de manière à pouvoir être extraite dans la direction axiale.

2. Instrument selon la revendication 1, caractérisé en ce que l'aiguille électrode (11) sert d'électrode de dissection monopolaire, une électrode neutre étant appliquée sur la peau du patient pour servir de contre-électrode.

3. Instrument selon la revendication 1, caractérisé en ce que la surface frontale (14) de la douille (12) sert d'électrode de coagulation monopolaire, une électrode neutre étant appliquée sur la peau du patient pour servir de contre-électrode.

4. Instrument selon la revendication 1, caractérisé en ce que l'aiguille électrode (11) sert d'électrode de dissection, et que la surface frontale (14) de la douille (12) sert d'électrode neutre.

5. Instrument selon l'une quelconque des revendications précédentes, caractérisé en ce que la surface frontale (14) de la douille (12) a une forme convexe.

6. Instrument selon l'une quelconque des revendications précédentes, caractérisé en ce qu'un actionnement (17) est disposé de préférence à l'extrémité proximale de l'élément de tige (13) afin de rentrer ou de sortir l'aiguille électrode (11).

7. Instrument selon la revendication 6, caractérisé en ce que l'actionnement (17) permettant de rentrer ou de sortir l'aiguille électrode (11) dans ou hors de la douille (12) est électromagnétique.

8. Instrument selon la revendication 6, caractérisé en ce que l'actionnement (17) permettant rentrer ou de sortir l'aiguille électrode (11) dans ou hors de la douille (12) est pneumatique ou hydraulique.

9. Instrument selon la revendication 6, caractérisé en ce que ledit instrument est muni d'un dispositif mécanique (17) destiné à bloquer l'aiguille électrode (11) en position rentrée (figure 3b) ou sortie (figure 3a).

10. Instrument selon l'une quelconque des revendications précédentes, caractérisé en ce que la longueur de l'aiguille électrode (11) pouvant sortir hors de la douille (12) est réglable.

11. Instrument selon l'une quelconque des revendications précédentes, caractérisé en ce que l'aiguille électrode a un diamètre compris entre 0,1 et 0,5 millimètre.

12. Instrument selon l'une quelconque des revendications précédentes, caractérisé en ce que l'élément de tige peut être cintré de manière plastique.

13. Instrument selon l'une quelconque des revendications précédentes, caractérisé en ce que l'élément de tige a une longueur comprise entre 20 et 50 cm.

14. Instrument selon l'une quelconque des revendications précédentes, caractérisé en ce que la douille (12) et l'élément de tige sont d'un seul tenant.

15. Instrument selon l'une quelconque des revendications précédentes, caractérisé en ce que la douille (12) et l'élément de tige ont le même diamètre extérieur.

16. Instrument selon l'une quelconque des revendications précédentes, caractérisé en ce que le diamètre extérieur de la douille (12) au voisinage de la surface frontale (14) est plus petit que le diamètre extérieur de l'élément de tige.

17. Instrument selon la revendication 16, caractérisé en ce que le diamètre extérieur de l'élément de tige est compris entre 5 et 10 mm et le diamètre extérieur de la surface frontale (14) de la douille (12) est compris entre 2 et 5 mm.

18. Instrument selon la revendication 16, caractérisé en ce que le diamètre extérieur de l'élément de tige est réduit au moins sur un étage ou bien de manière conique à un diamètre extérieur plus petit de la surface frontale (14) de la douille (12).
